# FASCICULE DE BREVET EUROPEEN

(11) **EP 1 680 413 B1**
(45) Date de publication et mention de la délivrance du brevet: **19.11.2008**
(21) Numéro de dépôt: 04805281.5
(22) Date de dépôt: 22.10.2004
(51) Int. Cl.: C07D 317/18

(54) **NOUVEAU LIGAND ACTIVATEUR DES RECEPTEURS RAR-BETA, SON PROCEDE DE PREPARATION ET SON UTILISATION EN MEDECINE HUMAINE AINSI QU´ en COSMETIQUE.**
NEUER DEN RAR-beta-REZEPTOR AKTIVIERENDER LIGAND, VERFAHREN ZU DESSEN HERSTELLUNG UND DESSEN VERWENDUNG IN DER HUMANMEDIZIN UND DER KOSMETIK
NEW RAR-beta RECEPTOR-ACTIVATING LIGAND, METHOD FOR THE PREPARATION THEREOF AND USE THEREOF IN HUMAN MEDICINE AND IN COSMETICS

(30) Priorité: 23.10.2003 FR 0312410
(43) Date de publication de la demande: 19.07.2006
(73) Titulaire: GALDERMA RESEARCH & DEVELOPMENT, 06410 Biot (FR)
(72) Inventeur: COLLETTE, Pascal, F-06110 Le Cannet (FR)
(74) Mandataire: Allab, Myriam
(86) Numéro de dépôt international: PCT/FR2004/002718
(87) Numéro de publication internationale: WO 2005/040148

(56) Documents cités:
- CHARPENTIER B ET AL: "SYNTHESIS, STRUCTURE-AFFINITY RELATIONSHIPS, AND BIOLOGICAL ACTIVITIES OF LIGANDS BINDING TO RETINOIC ACID RECEPTOR SUBTYPES" JOURNAL OF MEDICINAL CHEMISTRY, AMERICAN CHEMICAL SOCIETY. WASHINGTON, US, vol. 38, no. 26, 1995, pages 4993-5006, XP000971461 ISSN: 0022-2623

## Description

L'invention se rapporte, à titre de produit industriel nouveau et utile, à un nouveau composé, ligand activateur des récepteurs RARbéta. Elle se rapporte également à son procédé de préparation et à son utilisation dans des compositions pharmaceutiques destinées à un usage en médecine humaine ou vétérinaire, ou bien encore dans des compositions cosmétiques.

Les composés ayant une activité de type rétinoïde (vitamine A et ses dérivés) sont largement décrits dans la littérature, comme ayant des activités dans les processus de prolifération et différentiation cellulaire. Ces propriétés confèrent à cette classe de composés, un fort potentiel dans le traitement ou la prévention de nombreuses pathologies, et plus particulièrement en dermatologie et dans le traitement des cancers. Beaucoup d'effets biologiques des rétinoïdes sont médiés par la modulation des récepteurs nucléaires de l'acide rétinoique (RAR).
Les récepteurs RARs activent la transcription en se liant à des éléments de séquences d'ADN, appelés les éléments de réponse des RAR Element (RARE), sous forme d'un hétérodimère avec les récepteurs X des rétinoïdes (appelés les RXRs).
Trois sous-types de RARs humains ont été identifiés et décrits : les RARα, RARβ et RARγ.

L'art antérieur contient un grand nombre de composés chimiques ayant une activité activatrice des récepteurs de type RAR. Parmi les documents de l'art antérieur, on peut citer plus particulièrement les composés bicycliques aromatiques décrits dans le brevet EP 0 722 928, des composés hétérocycliques aromatiques décrits dans le brevet EP 0 732 328, des composés biaryles hétérocycliques décrits dans la demande WO 97/29100, des composés propynyl biaromatiques décrits dans la demande WO 97/33856, des composés biaryl hétérocycliques aromatiques décrits dans le brevet EP 0 816 352.
Charpentiers et. al., Am. Chem. Soc., 38, (26), 1995, 4993-5006 seulement, decrit des composés n'ayant pas une structure dioxolanique comme activateur des récepteurs RAR-béta.

La Demanderesse a inventé un nouveau composé, qui de manière surprenante, a la particularité d'être un ligand activateur spécifique des récepteurs de l'acide rétinoique de sous type béta.

Ainsi, la présente invention concerne un composé répondant à la formule suivante : 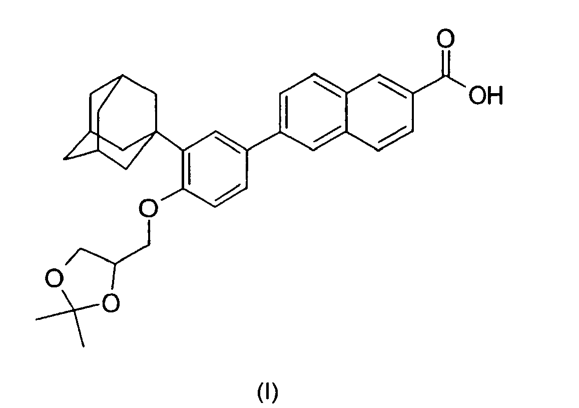 et les sels du composé de formule (I) ainsi que les isomères optiques et géométriques dudit composé de formule (I)**.**

Lorsque le composé selon l'invention se présente sous forme d'un sel, il s'agit de préférence d'un sel d'un métal alcalin ou alcalino-terreux, ou encore de zinc ou d'une amine organique.

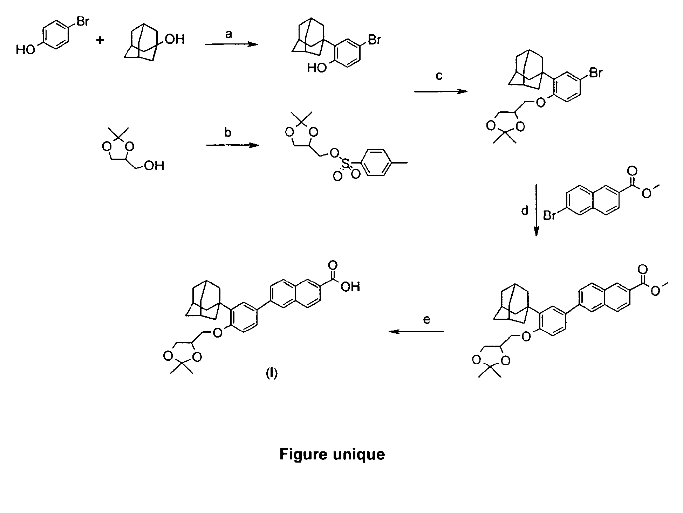

La présente invention a également pour objet le procédé de préparation du composé de formule (I), en particulier selon le schéma réactionnel donné à la Figure annexée.
a) le 2-adamantyl-4-bromophenol peut être obtenu par substitution électrophile de l'adamantanol sur le 4-bromophenol en présence d'acide sulfurique H₂SO₄ ;
b) l'alcool primaire de l'1,2-isopropylidene glycérol est activé par exemple par introduction d'un groupement tosyle tel que chlorure de tosyle TsCl en présence de pyridine ;
c) puis le composé obtenu est substitué sur le groupement phénol du 2-adamantyl-4-bromophenol décrit ci dessus, par exemple en présence de carbonate de potassium K₂CO₃, et de diméthylformamide DMF ;
d) ce bromure est alors couplé avec le 6-bromo-2-naphtoate de méthyle, par exemple dans des conditions de couplage de type Negishi (avec (i) Mg, tétrahydrofurane THF, (ii) ZnCl2, (iii) Ar-Br, NiCl2, Diphophinopropane éthylène Dppe), pour obtenir le 6-[3-adamantan-1-yl-4-(2,2-dimethyl-[1,3]dioxolan-4-ylmethoxy)-phenyl]-naphthalene-2-carboxylate de méthyle ;
e) enfin, une saponification, par exemple en présence d'hydroxyde de sodium NaOH permet d'obtenir l'acide 6-[3-Adamantan-1-yl-4-(2,2-dimethyl-[1,3]dioxolan-4-ylmethoxy)-phényl]-naphthalene-2-carboxylique.

Les composés selon l'invention présentent des propriétés activatrices des récepteurs de type RARbéta. Cette activité activatrice des récepteurs RARbéta peut notamment être mesurée dans un test de transactivation par la constante de dissociation Kdapp (apparent), l'AC50 (concentration donnant 50% de l'activité de la molécule de référence) et également par le % d'activation.
Par activateur des récepteurs de type RARbéta, on entend selon l'invention tout composé qui pour le sous-type RARbeta présente une constante de dissociation Kdapp inférieure ou égale à 1 µM, et une AC50 ≤ 100 nM, dans un test de transactivation tel que décrit dans l'exemple 2.
Le composé selon l'invention est un activateur des récepteurs de type RAR-β spécifique, c'est à dire qu'il présente un rapport R de Kd app relatif au RAR-β sur le Kdapp relatif au RAR-α ou RAR-γ inférieur ou égal à 10⁻¹. De préférence, R est inférieur ou égal à 0,05, et plus avantageusement inférieur ou égale à 0,02.

La présente invention a également pour objet le composé de formule (I) tel que décrit ci-dessus pour une utilisation comme médicament.

Le composé selon l'invention convient particulièrement bien dans les domaines de traitement suivants :
1) pour traiter les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal) ;
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telle que les kératoacanthomes ;
5) pour traiter d'autres désordres dermatologiques tels que les dermatoses immunes telles le lupus érythémateux, les maladies immunes bulleuses et les maladies du collagène, telle la sclérodermie ;
6) dans le traitement d'affections dermatologiques ou générales à composante immunologique ;
7) pour traiter certains troubles ophtalmologiques, notamment les cornéopathies,
8) pour prévenir ou guérir les stigmates de l'atrophie épidermique et/ou dermique induite par les corticostéroïdes locaux ou systémiques, ou toute autre forme d'atrophie cutanée,
9) dans le traitement de toute affection d'origine virale au niveau cutané ou général,
10) dans le traitement de désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
11) pour lutter contre les troubles de la fonction sébacée tels que l'hyperséborrhée de l'acné ou la séborrhée simple ;
12) pour prévenir ou traiter les troubles de la cicatrisation, ou pour prévenir ou pour réparer les vergetures, ou encore pour favoriser la cicatrisation,
13) dans le traitement des désordres de la pigmentation, tel l'hyperpigmentation, le mélasma, l'hypopigmentation ou le vitiligo ;
14) dans le traitement des affections du métabolisme des lipides, tel l'obésité, l'hyperlipidémie, ou le diabète non insulino-dépendant ;
15) dans le traitement d'affections inflammatoires telles que l'arthrite ;
16) dans le traitement ou la prévention des états cancéreux ou précancéreux ;
17) dans la prévention ou le traitement de l'alopécie de différentes origines, notamment l'alopécie due à la chimiothérapie ou aux rayonnements ;
18) dans le traitement des troubles du système immunitaire, tel l'asthme, le diabète sucré de type I, la sclérose en plaque, ou autres disfonctionnements sélectifs du système immunitaire ; et
19) dans le traitement d'affections du système cardiovasculaire telles que l'artériosclérose ou l'hypertension.

La présente invention a également pour objet une composition pharmaceutique comprenant, dans un milieu physiologiquement acceptable, au moins un composé de formule (I) tel que défini ci-dessus.
La présente invention a aussi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, le composé de formule (I), l'un de ses isomères optiques ou un de ses sels.

L'administration de la composition selon l'invention peut être effectuée par voie orale, entérale, parentérale, topique ou oculaire. De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Le composé selon l'invention est généralement administré à une dose journalière d'environ 0,01 mg/kg à 100 mg/kg de poids corporel, en 1 à 3 prises.

Le composé est utilisé par voie systémique à une concentration généralement comprise entre 0,001% et 10% en poids, de préférence entre 0,01% et 1% en poids, par rapport au poids de la composition.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

Le composé est utilisé par voie topique à une concentration généralement comprise entre 0,001% et 10% en poids, de préférence entre 0,01% et 1% en poids, par rapport au poids total de la composition.

Le composé de formule (I) selon l'invention trouve également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

L'invention a donc également pour objet une composition cosmétique comprenant, dans un support physiologiquement acceptable, le composé de formule (I).

Par un milieu physiologiquement acceptable, on entend un milieu compatible avec la peau et éventuellement avec ses phanères (cils, ongles, cheveux) et/ou les muqueuses.

L'invention a également pour objet l'utilisation non thérapeutique d'une composition cosmétique comprenant le composé de formule (I) pour prévenir et/ou traiter les signes du vieillissement et/ou la peau sèche.

L'invention a aussi pour objet l'utilisation non thérapeutique d'une composition cosmétique comprenant le composé de formule (I) pour l'hygiène corporelle ou capillaire.

La composition cosmétique selon l'invention contenant, dans un support cosmétiquement acceptable, le composé de formule (I) ou l'un de ses isomères optiques ou géométriques ou l'un de ses sels, peut se présenter notamment sous forme d'une crème, d'un lait, d'un gel, de suspensions de microsphères ou nanosphères ou vésicules lipidiques ou polymériques, de tampons imbibés, de solutions, de sprays, de mousses, de sticks, de savons, de bases lavantes ou de shampooings.

La concentration en composé de formule (I) dans la composition cosmétique est de préférence comprise entre 0,001% et 3% en poids, par rapport au poids total de la composition.

Les compositions pharmaceutiques et cosmétiques telles que décrites précédemment peuvent en outre contenir des additifs inertes, ou même pharmacodynamiquement actifs pour ce qui concerne les compositions pharmaceutiques, ou des combinaisons de ces additifs, et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des antioxydants,
- des émollients ;
- des agents hydratants ;
- des agents antiséborrhéiques ou antiacnéiques, ;
- des antibiotiques ;
- des agents antifongiques ;
- des agents favorisant la repousse des cheveux,;
- des agents anti-inflammatoires non stéroïdiens ;
- des caroténoïdes et, notamment, le β-carotène ;
- des agents anti-psoriatiques;
- des acides eicosa-5,8,11,14-tétraynoïque et eicosa-5,8,11-triynoïque, leurs esters et amides ;
- des rétinoïdes, c'est à dire des ligands des récepteurs RXR, naturels ou synthétiques ;
- des corticostéroïdes ou des oestrogènes;
- des α-hydroxy acides et des α-céto acides ou leurs dérivés, ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les cytokines ou les facteurs de croissance...).
   Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.
   L'invention porte également sur un procédé cosmétique d'embellissement de la peau,
caractérisé par le fait que l'on applique sur la peau une composition cosmétique telle que définie précédemment.

On va maintenant donner, à titre d'illustration et sans aucun caractère limitatif, un exemple d'obtention du composé actif de formule (I) selon l'invention, des résultats d'activité biologique ainsi que diverses formulations concrètes à base du composé.

La Figure représente le schéma réactionnel d'un procédé de préparation du composé de formule (I).

### Exemple 1 : Acide 6-[3-Adamantan-1-yl-4-(2,2-dimethyl-[1,3]dioxolan-4-ylmethoxy)-phényl]-naphthalene-2-carboxylique

### 1. Toluene-4-sulfonate de 2,2-dimethy-[1,3]dioxolan-4-ylmethyle

57,2g (300 mmol) de chlorure de tosyle sont ajoutés à une solution de 39,6g (300 mmol) de (2,2-dimethyl-[1,3]dioxolan-4-yl)-méthanol dans 170 ml de pyridine a 0°C. Le mélange est agité pendant une nuit, dilué par de l'éther, lavé par une solution d'acide chlorhydrique 1 N jusqu'a ce que la phase aqueuse soit à pH acide, puis lavé par une solution saturée d'hydrogénocarbonate de sodium.
La phase organique est alors séchée sur sulfate de sodium, filtrée et évaporée. Une huile incolore est obtenue (m = 79,3g , rendement = 92%).

### 2. 2-Adamantan-1-yl-4-bromo-phenol

51,9g (300mmol) de 4-bromo-phenol, 45,7g (300mmol) d'adamantan-1-ol sont dissous dans 150 mL de dichlorométhane ; 15ml d'acide sulfurique 98% sont coulés lentement et le milieu réactionnel est agité à température ambiante pendant deux jours. Le milieu est évaporé a sec, repris par de l'eau, neutralisé a pH=7 - 8, filtré, repris dans THF, séché sur MgSO4, évaporé et purifié sur colonne de silice éluée par un mélange éther/heptane 10/90. Une huile épaisse est obtenue (m = 58g, rendement = 63%).

### 3. 4-(2-Adamantan-1-yl-4-bromo-phenoxymethyl)-2,2-dimethyl-[1,3]dioxolane

### 10g (32,5 mmol) de 2-adamantan-1-yl-4-bromo-phenol, 4,95g (35,8 mmol) de carbonate de potassium sont mélangés dans 140 ml de DMF.

Une solution de toluene-4-sulfonate de 2,2-dimethy-[1,3]dioxolan-4-ylmethyle (11,2g, 39 mmol) dans 60 mL de DMF est coulée goutte à goutte sur le milieu réactionnel tout en chauffant ce dernier à 100°C. Le milieu réactionnel est agité à 100°C pendant 24h. Le milieu réactionnel est versé sur de l'eau glacée et extrait par de l'éther. La phase organique est lavée par l'eau puis par la saumure, séchée sur MgSO4, filtrée et évaporée. Les 14 g de produit brut obtenus sont purifiés sur colonne de silice fine (6-35 µm) avec une élution par un mélange dichlorométhane/heptane 50/50.
Après évaporation des fractions, 7,51g (55%) d'une poudre jaune sont obtenus (point de fusion pf: 91°C).

### 4. 6-[3-Adamantan-1-yl-4-(2,2-dimethyl-[1,3]dioxolan-4-ylmethoxy)-phényl]-naphthalene-2-carboxylate de méthyle

962 mg (39,6 mmol) de magnésium en granules sont mouillés par un minimum de THF. Une solution de 7,4g (17,6 mmol) de 4-(2-adamantan-1-yl-4-bromo-phenoxymethyl)-2,2-dimethyl-[1,3]dioxolane, 50 ml de THF et 300µl (3,5mmol)de dibromoéthane est coulée goutte à goutte sur la suspension de magnésium. Le milieu réactionnel est chauffé à reflux du THF pendant 1h30 puis ramené à 20°C. 5,1 g (37,6mmol) de chlorure de zinc préalablement séché et fondu sous vide sont ajoutés en conservant la température du milieu à 20°C, puis 50ml de THF sont ajoutés et le milieu est agité à 20°C pendant 1 h30. 10g (37,6mmol) de 6-bromo-naphthalene-2-carboxylate de méthyle sont ajoutés par petites fractions en maintenant la température du milieu à 20°C, puis 300mg de complexe NiCl2.dppe et 50 ml de THF sont ajoutés et le milieu réactionnel est agité à température ambiante pendant 15h. Le milieu réactionnel est alors traité par une solution saturée de chlorure d'ammonium et extrait à l'éther. La phase organique est filtrée, lavée à l'eau puis séchée et concentrée sous pression réduite. Le résidu obtenu est alors purifié par chromatographie sur colonne de silice (éluant Heptane 70 / dichlorométhane 30). Un solide blanc est obtenu (m = 8,3 g, rendement = 89%, pf = 117°C).

### 5. Acide 6-[3-Adamantan-1-yl-4-(2,2-dimethyl-[1,3]dioxolan-4-ylmethoxy)-phényl]-naphthalene-2-carboxylique

A 1g (1,9mmol) de 6-[3-adamantan-1-yl-4-(2,2-dimethyl-[1,3]dioxolan-4-ylmethoxy)-phényl]-naphthalene-2-carboxylate de méthyle sont ajoutés 2g (50 mmol) d'hydroxyde de sodium et 25ml de méthanol (soit 25 ml d'une solution à 2N de soude méthanolique). Le milieu réactionnel est chauffé à reflux du méthanol pendant 6h puis 2 jours à température ambiante et enfin évaporé a sec. Le résidu solide est repris dans l'eau,la phase aqueuse est acidifiée à pH=2-3 et extraite par de l'éther. La phase organique est lavée par l'eau puis par de la saumure, séchée sur sulfate de magnésium, filtrée et évaporée jusqu'a ce qu'il ne reste qu'environ 10 ml d'une suspension du produit dans l'éther. Les cristaux sont alors filtrés et séchés. On obtient après séchage 862mg (88%) d'une poudre jaunâtre (pf = 287°C). RMN ¹H (CDCl₃ + gtte D6 DMSO) (250 Mhz): 1.43(s,3H), 1.49(s,3H), 1.80(s,6H), 2.12(s broad, 3H), 2.18(s,6H), 4.05(m, 2H), 4.16-4.30(m, 2H), 4.60(m, 1 H), 7.0 (d, J=8.5Hz, 1H), 7.55(d, J=8.5Hz, 1H), 7.6(s, 1 H), 7.78(d, J=8.5Hz, 1 H), 7.89-8.00(m, 3H), 8.09(d, J=10.0Hz, 1 H), 8.63(s, 1H).

### Exemple 2 : Test de transactivation

L'activation des récepteurs par un agoniste (activateur) dans des cellules HeLa conduit à l'expression d'un gène reporter, la luciférase, qui, en présence d'un substrat génère de la lumière. On peut donc mesurer l'activation des récepteurs en quantifiant la luminescence produite après incubation des cellules en présence d'un antagoniste de référence. Les produits activateurs vont déplacer l'antagoniste de son site permettant ainsi l'activation du récepteur. La mesure de l'activité se fait par la quantification de l'augmentation de la lumière produite. Cette mesure permet de déterminer l'activité activatrice du composé selon l'invention.

Dans cette étude, nous déterminons une constante qui représente l'affinité de la molécule pour le récepteur. Cette valeur pouvant fluctuer selon l'activité basale et l'expression du récepteur, on la dénomine Kd apparent (KdApp).
Pour déterminer cette constante, des « courbes croisées » du produit à tester contre un antagoniste de référence autrement nommé ligand de référence, l'acide 4-(5,5-Dimethyl-8-p-tolyl-5,6-dihydro-naphthalen-2-ylethynyl)-benzoïque sont réalisées en plaque 96 puits. Le produit à tester est utilisé à 10 concentrations et l'antagoniste de référence à 7 concentrations. Dans chaque puit, les cellules sont en contact d'une concentration du produit à tester et d'une concentration de l'antagoniste de référence, l'acide 4-(5,5-Dimethyl-8-p-tolyl-5,6-dihydro-naphthalen-2-ylethynyl)-benzoique.
Des mesures sont également réalisées pour les témoins agoniste total autrement nommé témoin 100% (l'acide 4-[2-(5,5,8,8 tetramethyl-5,6,7,8 tetrahydronaphtalene-2-yl)propenyl]-benzoique) et agoniste inverse autrement nommé témoin 0%, l'acide 4-{(E)-3-[4-(4-tert-Butyl-phenyl)-5,5,8,8-tetramethyl-5,6,7,8-tetrahydro-naphthalen-2-yl]-3-oxo-propenyl}-benzoïque.
Ces courbes croisées nous permettent de déterminer les AC50 (concentration à laquelle on observe 50% d'activation) du ligand de référence à différentes concentrations de produit à tester. Ces AC50 sont utilisées pour calculer la régression de Schild en traçant une droite répondant à l'équation de Schild (« quantitation in receptor pharmacology » Terry P.Kenakin, Receptors and Channels, 2001,7, 371-385).
Dans le cas d'un agoniste, nous calculons une AC50 (concentration donnant 50% de l'activité) en traçant la courbe du produit à la concentration du ligand de référence donnant 80% d'activation. Nous mesurons également le % d'activation qui correspond au niveau maximum d'activité obtenue.

Les lignées cellulaires HeLa utilisées sont des transfectants stables contenant les plasmides ERE-βGlob-Luc-SV-Neo (gène reporter) et RAR (α, β, γ) ER-DBD-puro. Ces cellules sont ensemencées en plaques 96 puits à raison de 10 000 cellules par puit dans 100µl de milieu DMEM sans rouge de phénol et supplémenté par 10% de sérum de veau délipidé. Les plaques sont ensuite incubées à 37°C, 7% CO₂ pour 4 H.
Les différentes dilutions du produit à tester, du ligand de référence (l'acide 4-(5,5-dimethyl-8-p-tolyl-5,6-dihydro-naphthalen-2-ylethynyl)-benzoique), du témoin 100% (l'acide 4-[2-(5,5,8,8 tetramethyl-5,6,7,8 tetrahydronaphtalene-2-yl)propenyl]-benzoïque 100 nM) et du témoin 0% (l'acide 4-{(E)-3-[4-(4-tert-butyl-phenyl)-5,5,8,8-tetramethyl-5,6,7,8-tetrahydro-naphthalen-2-yl]-3-oxo-propenyl}-benzoïque 500 nM) sont rajoutées à raison de 5 µl par puits. Les plaques sont ensuite incubées 18 heures à 37°C, 7% CO₂.
Le milieu de culture est éliminé par retournement et 100 µl d'un mélange 1 :1 PBS/Luciferine est ajouté à chaque puit. Après 5 minutes, les plaques sont lues par le lecteur de luminescence.

| | RARalpha | | | RARbeta | | | RARgamma | | |
|---|---|---|---|---|---|---|---|---|---|
| | Kd app (nM) | AC50 (nM) | % d'activation | Kd app (nM) | AC50 (nM) | % d'activation | Kd app (nM) | AC50 (nM) | % d'activation |
| Ex1 | 8000 | 1800 | 20 | 60 | 12 | 100 | 4000 | 2000 | 40 |

Les résultats obtenus avec le composé selon l'invention montrent bien une grande spécificité du composé selon l'invention pour le sous-type de récepteur RARbéta en comparaison avec les deux autres sous-types RARalpha et RARgamma (rapport des Kd app R= 0.015), ceci démontrant bien une augmentation du signal, de la luminescence en présence de l'antagoniste de référence. Le composé selon l'invention est donc bien activateur sélectif des récepteurs RARbéta.

### Exemple 3 : exemples de formulation

Dans cet exemple, on a illustré diverses formulations concrètes à base des composés selon l'invention.

### A- VOIE ORALE

(a) Comprimé de 0,2 g
   - Composé de l'exemple 1 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 1 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 1 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10ml
   - Composé de l'exemple 1 0,200 g
   - Glycérine 1,000 g
   - Sorbitol à 70% 1,000 g
   - Saccharinate de sodium 0,010 g
   - Parahydroxybenzoate de méthyle 0,080 g
   - Arome qs
   - Eau purifiée qsp 10 ml

### B- VOIE PARENTERALE

(a) Composition
   - Composé de l'exemple 1 0,002 g
   - Oléate d'éthyle qs 10 g
(b) Composition
   - Composé de l'exemple 1 0.05 %
   - Polyéthylène glycol 20%
   - Solution de NaCl à 0.9% qs 100
(c) Composition
   - Composé de l'exemple 1 2.5 %
   - Polyéthylène glycol 400 20%
   - Solution de NaCl à 0.9% qs 100
(d) Composition de cyclodextrine injectable
   Composé de l'exemple 1 0,1mg
   β- cyclodextrine 0,10 g
   Eau pour injectable q.s.p.10,00 g

### C- VOIE TOPIQUE

(a) Onguent
   - Composé de l'exemple 1 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 1 0,300 g
   - Vaseline blanche codex qsp 100 g
(c) Crème Eau-dans-Huile non ionique
   - Composé de l'exemple 1 0,100 g
   - Mélange d'alcools de lanoline émulsifs, de cires
      et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 1 0,100 g
   - Polyéthylène glycol (PEG 400) 69,900 g
   - Ethanol à 95% 30,000 g
(f) Crème Huile-dans-Eau non ionique
   - Composé de l'exemple 1 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Composé **caractérisé par le fait qu'**il répond à la formule (I) suivante : 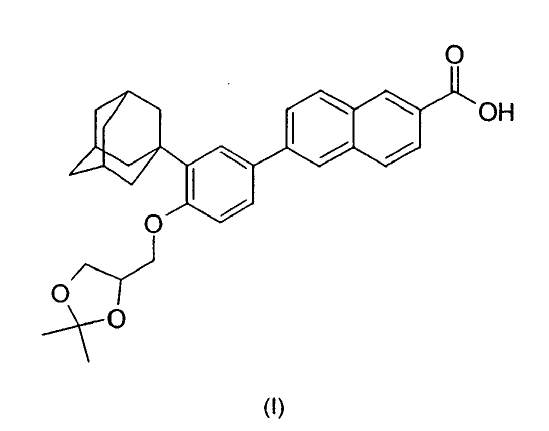 et ses sels ainsi que les isomères optiques et géométriques dudit composé de formule (I).

2. Composé selon la revendication 1, **caractérisé par le fait qu'**il se présente sous forme de sels d'un métal alcalin ou alcalino-terreux, de sels de zinc, ou de sels d'une amine organique.

3. Composé selon l'une des revendications 1 ou 2 pour une utilisation comme médicament.

4. Utilisation d'un composé selon l'une des revendications 1 ou 2 dans la fabrication d'une composition pharmaceutique destinée au traitement :
- des affections dermatologiques liées à un désordre de la kératinisation portant sur la différenciation et sur la prolifération cellulaire ;
- des ichtyoses, des états ichtyosiformes, de la maladie de Darrier, des kératodermies palmoplantaires, des leucoplasies et des états leucoplasiformes, du lichen cutané ou muqueux (buccal) ;
- des affections dermatologiques avec une composante immuno-allergique inflammatoire, avec ou sans trouble de la prolifération cellulaire ;
- des proliférations dermiques ou épidermiques bénignes ou malignes, d'origine virale ou non ;
- des proliférations pouvant être induites par les ultra-violets ;
- des lésions précancéreuses cutanées ;
- des dermatoses immunes ;
- des maladies immunes bulleuses ;
- des maladies du collagène ;
- des affections dermatologiques à composante immunologique ;
- des troubles ophtalmologiques;
- des stigmates de l'atrophie épidermique et/ou dermique induite par les corticostéroïdes locaux ou systémiques, ou toute autre forme d'atrophie cutanée;
- des affection d'origine virale au niveau cutané;
- de désordres cutanés dus à une exposition aux rayonnements U.V, du vieillissement de la peau, photo-induit ou chronologique ou des pigmentations et des kératoses actiniques ;
- des pathologies associées au vieillissement chronologique ou actinique de la peau ;
- des troubles de la fonction sébacée ;
- des troubles de la cicatrisation ou des vergetures ; ou
- des désordres de la pigmentation.

5. Composition pharmaceutique, **caractérisée par le fait qu'**elle comprend, dans un support physiologiquement acceptable, le composé tel que défini à l'une des revendications 1 ou 2.

6. Composition selon la revendication 5, **caractérisée en ce que** la concentration en composé selon l'une des revendications 1 ou 2 est comprise entre 0,001 % et 10% en poids par rapport au poids total de la composition.

7. Composition selon la revendication 6, **caractérisée en ce que** la concentration en composé selon l'une des revendications 1 ou 2 est comprise entre 0, 01% et 1% en poids par rapport au poids total de la composition.

8. Composition cosmétique, **caractérisée par le fait qu'**elle comprend, dans un support cosmétiquement acceptable, le composé tel que défini à l'une des revendications 1 ou 2.

9. Composition selon la revendication 8, **caractérisée en ce que** la concentration en composé selon l'une des revendications 1 ou 2 est comprise entre 0,001% et 3% en poids par rapport au poids total de la composition.

10. Utilisation non thérapeutique d'une composition cosmétique telle que définie à l'une des revendications 8 ou 9 pour prévenir et/ou traiter les signes du vieillissement et/ou la peau sèche.

11. Utilisation non thérapeutique d'une composition cosmétique telle que définie à l'une des revendications 8 ou 9 pour l'hygiène corporelle ou capillaire.

12. Procédé cosmétique d'embellissement de la peau, **caractérisé par le fait que** l'on applique sur la peau une composition telle que définie dans l'une quelconque des revendications 8 à 9.

## Claims

1. Compound **characterized in that** it corresponds to formula (I) below: 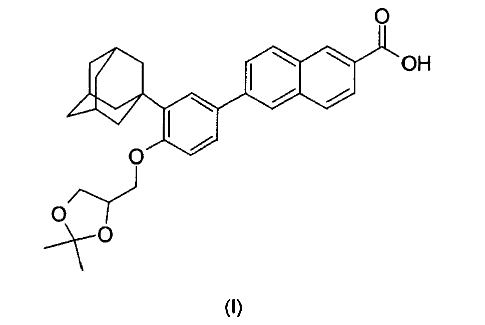 and its salts and also the optical and geometric isomers of said compound of formula (I).

2. Compound according to Claim 1, **characterized in that** it is in the form of alkali metal or alkaline-earth metal salts, zinc salts or salts of an organic amine.

3. Compound according to either of Claims 1 and 2, for use as a medicinal product.

4. Use of a compound according to either of Claims 1 and 2, in the production of a pharmaceutical composition intended for treating:
- dermatological complaints associated with a keratinization disorder relating to cell differentiation and proliferation;
- ichthyosis, ichthyosiform conditions, Darier's disease, palmoplantar keratoderma, leukoplakia and leukoplakiform conditions, and cutaneous or mucosal (buccal) lichen;
- dermatological complaints with an inflammatory immunoallergic component, with or without cell proliferation disorder;
- benign or malignant, dermal or epidermal proliferations of viral or nonviral origin;
- proliferations that may be induced by ultraviolet radiation;
- cutaneous precancerous lesions;
- immune dermatoses;
- immune bullous diseases;
- collagen diseases;
- dermatological complaints with an immunological component;
- ophthalmological disorders;
- stigmata of epidermal and/or dermal atrophy induced by local or systemic corticosteroids, or any other form of cutaneous atrophy;
- cutaneous complaints of viral origin;
- skin disorders caused by exposure to UV radiation, photoinduced or chronological ageing of the skin, or pigmentations and actinic keratosis;
- pathologies associated with chronological or actinic ageing of the skin;
- sebaceous function disorders;
- cicatrization disorders, or stretch marks; or
- pigmentation disorders.

5. Pharmaceutical composition, **characterized in that** it comprises, in a physiologically acceptable support, the compound as defined in either of Claims 1 and 2.

6. Composition according to Claim 5, **characterized in that** the concentration of compound according to either of Claims 1 and 2 is between 0.001% and 10% by weight, relative to the total weight of the composition.

7. Composition according to Claim 6, **characterized in that** the concentration of compound according to either of Claims 1 and 2 is between 0.01% and 1% by weight, relative to the total weight of the composition.

8. Cosmetic composition, **characterized in that** it comprises, in a cosmetically acceptable support, the compound as defined in either of Claims 1 and 2.

9. Composition according to Claim 8, **characterized in that** the concentration of compound according to either of Claims 1 and 2 is between 0.001% and 3% by weight, relative to the total weight of the composition.

10. Non-therapeutic use of a cosmetic composition as defined in either of Claims 8 and 9, for preventing and/or treating the signs of ageing and/or dry skin.

11. Non-therapeutic use of a cosmetic composition as defined in either of Claims 8 and 9 for body or hair hygiene.

12. Cosmetic process for making the skin more attractive, **characterized in that** a composition as defined in either one of Claims 8 and 9 is applied to the skin.

## Patentansprüche

1. Verbindung, **dadurch gekennzeichnet, dass** sie der folgenden Formel (I) entspricht: 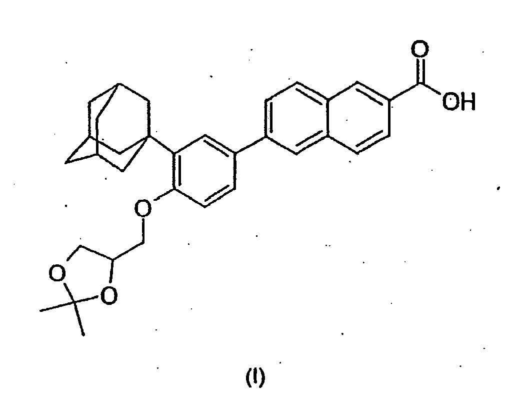 und ihre Salze sowie die optischen und geometrischen Isomere der Verbindung der Formel (I).

2. Verbindung nach Anspruch 1, **dadurch gekennzeichnet, dass** sie in Form der Alkalimetallsalze, Erdalkalimetallsalze, Zinksalze oder Salze eines organischen Amins vorliegt.

3. Verbindung nach einem der Ansprüche 1 oder 2 für eine Verwendung als Arzneimittel.

4. Verwendung einer Verbindung nach einem der Ansprüche 1 oder 2 bei der Herstellung einer pharmazeutischen Zusammensetzung, die vorgesehen ist für die Behandlung von:
- dermatologischen Erkrankungen, die mit einer Störung der Keratinisierung zusammenhängen, die auf der Differenzierung und der Proliferation der Zellen beruht;
- Ichthyosis, ichthyosiformen Zuständen, der Darier-Krankheit, Keratoma palmoplantaris, Leukoplasien und leukoplasiformen Zuständen, Lichen der Haut oder der Schleimhaut (buccal);
- dermatologischen Erkrankungen mit einer entzündlichen immunoallergischen Komponente, mit oder ohne Störungen der Zellproliferation;
- dermalen oder epidermalen Proliferationen, die benigne oder maligne und die gegebenenfalls viralen Ursprungs sind;
- Proliferationen, die durch UV-Strahlung induziert sein können;
- präkanzerösen Hautläsionen;
- Immundermatosen;
- bullösen Immunerkrankungen;
- Erkrankungen des Collagens;
- dermatologischen Erkrankungen mit immunologischer Komponente;
- ophthalmologischen Störungen;
- Stigmen epidermaler und/oder dermaler Atrophie, die durch lokale oder systemische Corticosteroide hervorgerufen wird,
- oder allen anderen Formen der Atrophie;
- Hauterkrankungen viralen Ursprungs;
- Hautstörungen, die durch Exposition gegenüber UV-Strahlung verursacht werden, lichtinduzierter oder altersbedingter Hautalterung oder Pigmentierungen und aktinischen Keratosen;
- Erkrankungen, die mit der altersbedingten oder aktinischen Hautalterung zusammenhängen;
- Funktionsstörungen der Talgdrüse;
- Störungen der Wundheilung oder Dehnungsstreifen; oder
- Pigmentierungsstörungen.

5. Pharmazeutische Zusammensetzung, **dadurch gekennzeichnet, dass** sie in einem physiologisch akzeptablen Träger die in einem der Ansprüche 1 oder 2 definierte Verbindung enthält.

6. Zusammensetzung nach Anspruch 5, **dadurch gekennzeichnet, dass** die Konzentration der Verbindung nach einem der Ansprüche 1 oder 2 im Bereich von 0,001 bis 10 Gew.-%, bezogen auf das Gesamtgewicht der Zusammensetzung, liegt.

7. Zusammensetzung nach Anspruch 6, **dadurch gekennzeichnet, dass** die Konzentration der Verbindung nach einem der Ansprüche 1 oder 2 im Bereich von 0,01 bis 1 Gew.-%, bezogen auf das Gesamtgewicht der Zusammensetzung, liegt.

8. Kosmetische Zusammensetzung, **dadurch gekennzeichnet, dass** sie in einem kosmetisch akzeptablen Träger die in einem der Ansprüche 1 oder 2 definierte Verbindung enthält.

9. Zusammensetzung nach Anspruch 8, **dadurch gekennzeichnet, dass** die Konzentration der Verbindung nach einem der Ansprüche 1 oder 2 im Bereich von 0,001 bis 3 Gew.-%, bezogen auf das Gesamtgewicht der Zusammensetzung, liegt.

10. Nichttherapeutische Verbindung einer kosmetischen Zusammensetzung nach einem der Ansprüche 8 oder 9, um den Anzeichen der Alterung und/oder trockener Haut vorzubeugen und/oder diese zu behandeln.

11. Nichttherapeutische Verwendung einer kosmetischen Zusammensetzung nach einem der Ansprüche 8 oder 9 für die Körper- oder Haarpflege.

12. Kosmetisches Verfahren zur Verschönerung der Haut, **dadurch gekennzeichnet, dass** auf die Haut eine Zusammensetzung aufgebracht wird, wie sie in einem der Ansprüche 8 oder 9 definiert ist.
